(19) **Europäisches Patentamt**

**European Patent Office**

**Office européen des brevets**

(11) **EP 0 955 976 B1**

(12) **EUROPEAN PATENT SPECIFICATION**

(45) Date of publication and mention
of the grant of the patent:
**20.03.2002 Bulletin 2002/12**

(51) Int Cl.[7]: **A61F 13/15**

(86) International application number:
**PCT/US95/09944**

(21) Application number: **95928328.4**

(22) Date of filing: **04.08.1995**

(87) International publication number:
**WO 96/08224 (21.03.1996 Gazette 1996/13)**

(54) **PARTIALLY ELASTIC, DISPOSABLE ABSORBENT PANT HAVING A SUBSTANTIALLY UNIFORMLY, FULLY GATHERED ELASTIC WAISTBORDER**

TEILWEISE ELASTISCHE WEGWERFWINDEL MIT EINEM GLEICHMÄSSIG FALTENDEN TAILLENBEREICH

CULOTTE ABSORBANTE JETABLE, PARTIELLEMENT ELASTIQUE DONT LA BORDURE DE CEINTURE ELASTIQUE EST TOTALEMENT ET PRATIQUEMENT UNIFORMEMENT FRONCEE

(84) Designated Contracting States:
**BE DE ES FR GB IT NL SE**

(30) Priority: **15.09.1994 US 306353**

(43) Date of publication of application:
**17.11.1999 Bulletin 1999/46**

(73) Proprietor: **KIMBERLY-CLARK WORLDWIDE, INC.**
**Neenah, Wisconsin 54956 (US)**

(72) Inventor: **CESCO-CANCIAN, Annamaria**
**Kimberly, WI 54136 (US)**

(74) Representative: **Diehl, Hermann, Dr. Dipl.-Phys.**
**DIEHL, GLÄSER, HILTL & PARTNER**
**Patentanwälte**
**Augustenstrasse 46**
**80333 München (DE)**

(56) References cited:
**EP-A- 0 526 868**        **EP-A- 0 547 497**
**EP-A- 0 688 550**        **WO-A-95/00096**

EP 0 955 976 B1

**Description**

Background of the Invention

The present invention relates to disposable absorbent pants, and more particularly to a partially elastic, disposable absorbent pant having a substantially uniformly, fully gathered elastic waistborder for improving fit and comfort.

In the last several years, disposable absorbent pants have become available for use by children in the potty-training stage, and have proved to be extremely popular with mothers and caretakers. One style of training pant comprises a bodyside liner, an outer cover, an absorbent medium between the liner and the outer cover, and side seams that bond portions of the side edges of the pant together to form waist and leg openings.

Another style of training pant has elastic side panels that fit against the hips of the child, and discrete front and back elastic waist strips adjacent the waist opening. The waist strips generally are spaced from the elastic side panels. These elastic side panels and waist elastics result in uneven or non-uniform gathering at the waist opening.

Although these discrete front and back elastic waist strips may provide some gathering, and even some gasketing, at the front and back of the pant, they are not entirely effective in providing the desired gathering, gasketing, and comfortable fit at the waist that would result in a pant that is aesthetically pleasing, waste containing, and comfortably fitting.

EP-A-0 526 868 discloses a disposable absorbent garment generally comprising a three- layered structure having a liquid-permeable topsheet, a liquid-impermeable backsheet, and some type of absorbent between the topsheet and backsheet.

A disposable absorbent training pant is disclosed comprising a main body having a front panel, a back panel, a crotch panel, and a pair of side panels from a waist opening and a pair of leg openings. A stretch-bonded laminate waistband and stretch-bonded laminate leg members are disposed at the waist opening and leg openings, respectively.

The absorbent pant comprises a non-stretchable T-shaped backsheet. The absorbent pant further comprises an absorbent medium disposed on a non-stretchable central strip, and a pair of stretchable front ear portions.

EP-A-0 547 497 discloses a disposable three dimensional garment comprising a full outer cover including an inner surface, an outer surface, a pair of side sections, and waist and leg openings. A pair of stretchable side members are provided on the inner surface of the outer cover at respective side sections to provide stretch to the outer cover. The stretchable side members have a stretch gradient such that the stretchability varies between the waist opening and a respective leg opening.

Furthermore, the absorbent garment comprises a stretchable waistband operatively joined to the waist opening. A vertex of each triangularly-shaped stretchable side member is generally adjacent to the stretchable waistband.

Thus, there still exists a need for a training pant having improved containment, comfort, and aesthetics.

Summary of the Invention

In one form of the invention there is provided a disposable garment comprising a pant body defining a waist opening and a pair of leg openings; and a partially elastic, continuous waistborder comprising elastic side segments and at least one non-elastic segment. A continuous, elastic waistband is joined to the partially elastic, continuous waistborder in a manner that fully gathers the waistborder. without substantially inhibiting elasticities of the elastic side segments

In another form of the present invention there is provided the above disposable garment wherein said continuous waistborder defines a continuous waist opening (24), wherein

said continuous elastic waistband (32) comprises a material that is capable of being temporarily elastically inhibited, and has an elasticity equal to or greater than an elasticity of said elastic segment (82), and wherein
said continuous elastic waistband (32) is joined to said continuous waistborder (30) in a temporarily elastically inhibited state.

In still another form of the present invention there is provided the above disposable absorbent garment further comprising

a topsheet, (34)
a backsheet (36) wherein the partially elastic waistborder (30) is comprised in the backsheet (36), and
an absorbent structure (28) disposed between said topsheet (34) and said backsheet (36).

Brief Description of the Drawings

The above-mentioned and other features of the present invention and the manner of attaining them will become more apparent, and the invention itself will be better understood by reference to the following description of the invention, taken in conjunction with the accompanying drawings, wherein:

Fig. 1 illustrates a front perspective view of a disposable absorbent training pant incorporating the principles of the present invention;.

Fig. 2 illustrates the training pant in Fig. 1 prior to being activated;

Fig. 3 illustrates the training pant in Fig. 1 in a partially disassembled, exploded and extended flat perspective view;

Figs. 4A-4D illustrate joining an elastic waistband to a pant body in accordance with the principles of the present invention; and

Figs. 5A-5E illustrate modifications of a cross-sectional view through a waist portion of the training pant in Fig. 1.

Definitions

Each of the following terms used herein includes the following meaning:

"Composite elastic" with reference to a material, substrate, or the like, refers to a multi-layered structure having at least one elastic layer joined to at least one gatherable layer at least at two locations wherein the gatherable layer is gathered between the locations where it is joined to the elastic layer. A composite elastic structure may be stretched to the extent that the non-elastic material gathered between the bond locations allows the elastic material to extend. This type of composite elastic structure is disclosed, for example, by Vander Wielen et al., U.S. Patent No. 4,720,415 issued January 19, 1988.

"Continuous" means that the described structure is a closed-loop structure. The continuous structure may be unitary, i.e., a one-piece structure, or may be made up of individual elements suitably joined together to form a closed-loop.

"Disposable" means that the described article is designed to be used until soiled, either by urination, defecation, or otherwise, and then discarded, rather than being washed and used again.

"Elastic", "elasticity", "elasticized", or the like, refers to a material or composite material that tends to recover its original size and shape after removal of the force causing the deformation, and is expressed as a percent.

"Elongation" means the ratio, expressed as a percent, of the extension of a material to the length of the material prior to the extension as represented by the following:

$$\frac{\text{extended length - original length}}{\text{original length}} \times 100.$$

"Extension", "extend", "extended", or variations thereof means the change in length of a material due to stretching, and is expressed in units of length.

"Extensible" means that the described material can be increased in length.

"Finished garment" , "finished pant", or the like, means that the garment or pant has been suitably manufactured for its intended use.

"Fully gathered" with reference to, for example, an opening or border means that the material about the opening or border is gathered along its total periphery.

"Join", "joining", "joined", or variations thereof, when used in describing the relationship between two or more elements, means that the elements can be connected together in any suitable manner, such as by heat sealing, ultrasonic bonding, thermal bonding, adhesives, stitching, or the like. Further, the elements can be joined directly together, or may have one or more elements interposed between them, all of which are suitably connected together.

"Operatively elastically joined" describes the joining of an elastic member to a non-elastic or partially elastic member such that the two joined members exhibit elasticity.

"Pant body" or "pant" refers to a garment that has a waist opening and a pair of leg openings, similar to shorts, swim wear, or the like. The described garment may or may not have manually tearable, non-refastenable side seams.

"Partially elastic" refers to a substrate, garment, a part of a garment, or the like, having at least one selected portion thereof that is elastic.

"Recover", "recovering", or variations thereof, refers to a contraction of an extended elastic material upon termination or removal of a biasing force, or upon suitably treating or activating the elastic material after it has been temporarily elastically inhibited.

"Substrate" means a layer which may be, for example, a film, a woven web, or a nonwoven web; or a composite structure comprising, for example, a topsheet, a backsheet, and an absorbent medium therebetween.

"Temporarily elastically inhibited", or variations thereof, means to delay the recovery of an extended elastic substrate or composite elastic material. The delay may be imparted by, for example, compressing the extended elastic substrate, or by, for example, compressing the composite elastic material so that the elastic and gatherable layers are temporarily joined. Partial recovery of a temporarily elastically inhibited elastic substrate or composite elastic material may occur immediately after the force is removed, but final recovery of such a temporarily elastically inhibited elastic substrate or composite elastic material will require more time than the recovery of the same material which has not been temporarily elastically inhibited. For example, recovery of an extended elastic substrate or composite elastic material that has not been temporarily elastically inhibited may be instantaneous, whereas the recovery of a temporarily elastically inhibited elastic substrate or composite elastic material may take, for example, from about 5 to about 60 seconds.

Detailed Description

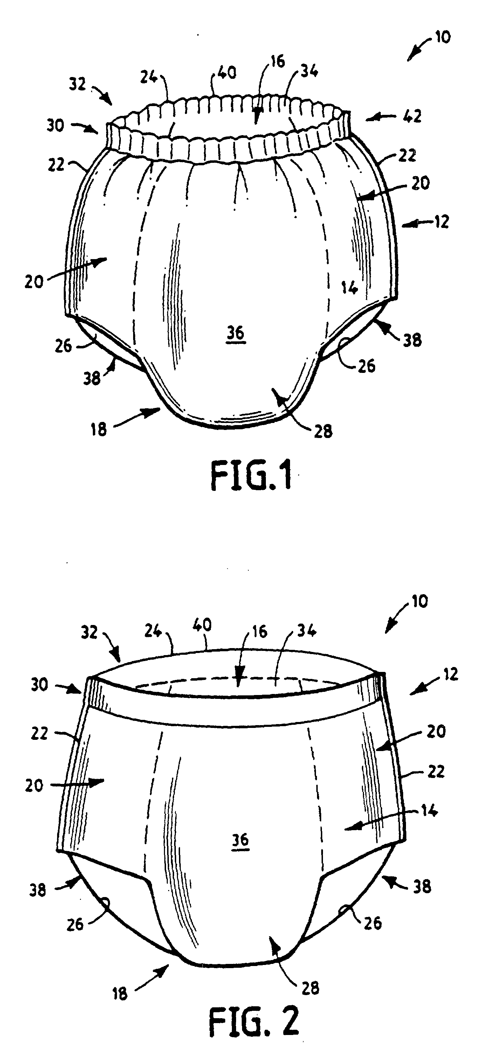

Referring primarily to Fig. 1, there is illustrated, in accordance with the principles of the present invention, disposable absorbent garment 10 in the form of a child's training pant. Although garment 10 is illustrated and will be described as a training pant, the present invention contemplates garment 10 including other pant-type products or the like.

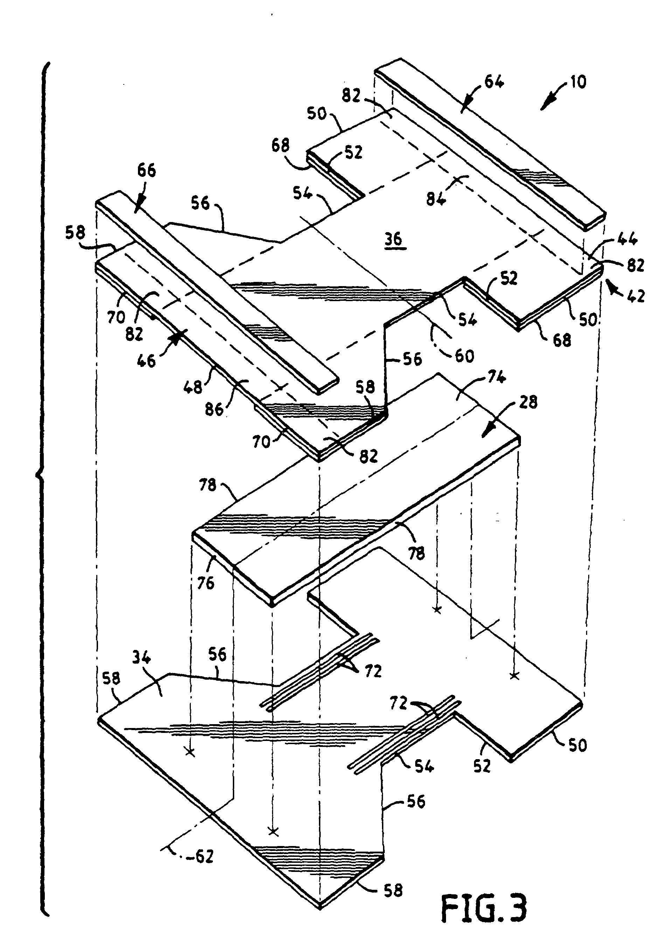

Disposable absorbent garment 10 includes a partially elastic pant body 12 comprising a front section 14, a back section 16, a crotch section 18, a pair of elastic side sections 20, a pair of seams 22, a continuous waist opening 24, and a pair of continuous leg openings 26. Each elastic side section 20 includes a front elastic side member 68 (Fig. 3) and a back elastic side member 70, which are joined together at a respective seam 22 (Fig. 1). Garment 10 further includes a fully gathered, continuous waistborder 30 (Fig. 1), continuous legborders 38, and a continuous waistband 32 operatively elastically joined to waistborder 30 of pant body 12.

Continuous waistband 32 comprises front waistband member 64 (Fig. 3) and back waistband member 66. Continuous waistborder 30 comprises front waistborder section 42 (Fig. 3) having front edge 44 and back waistborder section 46 having back edge 48. An absorbent structure 28 is suitably incorporated in any manner well-known in the art in garment 10 at least at crotch section 18 thereof.

Pant body 12 includes a topsheet 34 (Fig. 3) and a backsheet 36, which are desirably coincident with one another, although not a requirement of the present invention. Topsheet 34 and backsheet 36 (Fig. 3) include a respective pair of front outer edges 50, front inner edges 52, innermost edges 54, back sloping edges 56, and back outer edges 58.

Front waistband member 64 (Fig. 3) is suitably joined to front waistborder section 42, and back waistband member 66 is suitably joined to back waistborder section 46. Front and back waistborder sections 42, 46 desirably have respective lengths substantially corresponding to the respective relaxed lengths of front and back waistband members 64, 66, and widths substantially corresponding to the respective widths of front and back waistband members 64, 66; length being measured along a line generally parallel to transverse centerline 60 (Fig. 3) and width being measured along a line generally parallel to longitudinal centerline 62. A desired width range of waistband members 64, 66 is between about 1 centimeter to about 8 centimeters, and a more desired range is between about 2 centimeters to about 4 centimeters. In the case where, for example, one or both of the waistband members 64, 66 have some other geometric shape, i.e., not an elongate rectangular shape as illustrated in Fig. 3, each waistborder section 42, 46 desirably would have substantially the same geometric shape as its respective waistband member 64, 66.

When garment 10, in its flat, unfinished state during the manufacture thereof, is folded along a transverse fold-line, which is generally parallel to transverse centerline 60 (Fig. 3), and pairs of aligned front and back outer edges 50, 58 are joined together to form seams 22 (Fig. 1), the following construction is accomplished: (i) front waistborder section 42 and back waistborder section 46 form or define continuous waistborder 30; (ii) front waistband member 64 and back waistband member 66 form or define continuous waistband 32; (iii) a front inner edge 52, an innermost edge 54, and a back sloping edge 56 form or define a respective leg opening 26; (iv) and a front elastic side member 68 and a back elastic side member 70 form or define a respective elastic side section 20.

If desired, garment 10 may also comprise leg elastic members 72 (Fig. 3) that are suitably joined, for example, between topsheet 34 and backsheet 36 in an extended condition.

Absorbent structure 28 (Fig. 3) comprises front absorbent edge 74, back absorbent edge 76, and absorbent side edges 78. Front absorbent edge 74 and back absorbent edge 76 are respectively inboard of front waistband member 64 and back waistband member 66. Similarly, absorbent side edges 78 are inboard of respective leg elastic members 72. By "inboard" is meant that front absorbent edge 74, for example, is closer to transverse centerline 60 than front waistband member 64 is to centerline 60, and absorbent side edges 78 are closer to longitudinal centerline 62 than leg elastic members 72 are to centerline 62.

When garment 10 is properly fitted on the wearer, topsheet 34 faces toward the body of the wearer, and may or may not be the layer that directly contacts the skin. Topsheet 34 can be a liquid permeable, elastic or non-elastic, substantially hydrophobic material, such as a spunbonded web of synthetic polymer filaments. Topsheet 34 can also be a meltblown web or a bonded carded web of synthetic polymer filaments. Suitable synthetic polymers include, for example, polyethylene, polypropylene, and polyesters. Topsheet 34 has a pore size that readily allows the passage therethrough of liquids, such as urine and other body exudates. If desired, topsheet 34 can be treated with surfactants to selectively adjust its degree of wettability, and can also be selectively embossed or perforated with discrete slits or holes extending therethrough. Suitable topsheet materials can have a basis weight between about 10 grams per square meter (gsm) to about 26 gsm, and a thickness between about 0.013 centimeters to about 0.064 centimeters. The thickness of the topsheet material can be determined by employing an Ames Bulk Test (ASTM D-1777) performed at a restraining pressure of 0.2 psi (1.38 kPa).

Backsheet 36, which may or may not be the outermost layer of garment 10, can be liquid permeable or liquid impermeable, and may or may not have breathability, i.e., be vapor permeable. A suitable liquid permeable backsheet 36 is a nonwoven bicomponent web having a basis weight between about 15 gsm to about 50 gsm. The nonwoven bicomponent web may be a spunbond bicomponent web, or a bonded carded bicomponent web. Suitable bicomponent fibers are a wettable, polyethylene/polypropylene bicomponent fiber available from CHISSO Corporation, Osaka, Japan. In this particular bicomponent fiber, the polypropylene forms the core and the polyethylene forms the sheath of the fiber. Other fiber orientations are possible, such as multi-lobe, side-by-side or end-to-end. Another suitable liquid permeable material is a liquid permeable spunbond polypropylene nonwoven web having a basis weight between about 15 gsm to about 50 gsm.

A suitable liquid impermeable backsheet 36 is a 0.0015 centimeter polyethylene film from Edison Plastics Company, South Plainfield, New Jersey. Backsheet 36 can also be a two-ply laminate, in which the innermost layer can be the above-described liquid impermeable film or any other suitable liquid impermeable substrates, and the outermost layer can be the above-described liquid permeable spunbond polypropylene nonwoven web or any other suitable liquid permeable substrate. Backsheet 36 desirably has a thickness within the range of about 0.0013 to about 0.0051 centimeters.

Absorbent structure 28 can comprise any suitable absorbent material, natural or synthetic, or a combination thereof, along with superabsorbent material. The absorbent material of which absorbent structure 28 is made may also be encased in a tissue wrap (not shown) in order to maintain the integrity of the absorbent material comprising absorbent structure 28.

Suitable superabsorbent materials are available from various vendors, such as Dow Chemical Company, Hoechst-Celanese Corporation, and Allied Colloids, Inc. A suitable natural absorbent material is a wood pulp fluff identified by the trade designation CR1654 from Kimberly-Clark Corporation, Neenah, Wisconsin. One specific absorbent structure 28 that can be suitably used in garment 10 is described in US 5,509,915 (U.S. Patent Application Serial No. 08/096,654 filed July 22, 1993), inventors Hanson et al., which is assigned to the assignee of this application.

The construction of garment 10 can be accomplished in any conventional manner known in the art. For example, the structural elements can be joined together in any suitable manner, such as by heat sealing or ultrasonic bonding, or by adhering the elements together with a suitable adhesive. Suitable adhesives can be obtained from Findley Adhesives, Inc., Wauwatosa, Wisconsin, and can be applied in any manner, such as by spraying, slot-coat extrusion, printing, or the like. The applied adhesive can be in any desired configuration, such as continuous or discontinuous beads, continuous or discontinuous swirls, meltblown patterns, spray patterns, or the like.

Referring to Figs. 1-4D, front elastic side members 68 (Fig. 3) can have the same or different geometry from back elastic side members 70. Each front elastic side member 68 and each back elastic side member 70 is sandwiched between topsheet 34 and backsheet 36.

Elastic side members 68, 70 can be any suitable elastic material, one such material being a block copolymer of styrene-ethylbutadiene-styrene. Other types of materials are the KRATON® G series from The Shell Chemical Company such as KRATON® G-1650, KRATON® G-1652, KRATON® GX-1657 and KRATON® G-2740X. The KRATON® D series can also be used, as well as polyester elastomeric materials, polyurethane elastomeric materials, and polyamide elastomeric materials. Elastic side members 68, 70 can be a film, nonwoven web, or ribbons or threads of synthetic or natural rubber arranged, for example, in a spaced, parallel manner.

Still other examples of elastic materials and composites are described in U.S. Patent No. 4,720,415, issued January 19, 1988, to Vander Wielen et al.; U.S. Patent No. 4,657,802, issued April 14, 1987, to Morman; and U.S. Patent No. 4,652,487 issued March 24, 1987, to Morman.

Each elastic side members 68, 70 is operatively elastically joined to a portion of either topsheet 34 or backsheet 36, preferably to portions of both topsheet 34 and backsheet 36, in order to provide elasticity to those portions. One example of this is disclosed in EP 0 743 052 (U.S. Patent Application Serial No. 08/043,132 filed March 25, 1993), inventor Van Gompel et al., which is incorporated by reference herein. Another example of providing elastic side sections 20 is described in U.S. Patent No. 4,940,464, issued July 10, 1990, to inventor Van Gompel et al..

Each elastic side section 20 in a finished garment 10 (Fig. 1) has an elasticity between about 50% to about 250%, desirably an elasticity between about 100% to about 250%, and more desirably an elasticity between about 100% to about 200%.

Continuous waistborder 30 (Fig. 1) comprises oppositely disposed elastic side segments 82 (Fig. 3), a non-elastic front segment 84, and a non-elastic back segment 86. Thus, continuous waistborder 30 (Fig. 1) is partially elastic. Non-elastic front segment 84 and non-elastic back segment 86, as illustrated in Fig. 3, include portions of topsheet 34 and backsheet 36. Desirably, waistborder 30 has a width between about 1 centimeter to about 8 centimeters, and a more desired width between about 2 centimeters to about 4 centimeters.

Continuous waistband 32 (Fig. 1) can be an elastomeric, cloth-like, nonwoven fibrous material, such as an elastomeric stretch bonded laminate web or an elastomeric meltblown web. By proper selection of materials, continuous waistband 32 can be rendered temporarily elastically inhibited, such as by, for example, compression. Once temporarily elastically inhibited, the elastic material, of which waistband 32 is comprised, can be activated, such as by, for example, treating with heat, to recover to a state of elasticity.

In one specific embodiment, waistband 32 comprises an elastomeric nonwoven fibrous web that is substantially vapor-permeable. Examples of suitable elastomeric nonwoven fibrous webs are described in U.

S. Patent No. 4,663,220 issued May 5, 1987, to Wisneski et al.. Examples of composite fabrics comprising at least one layer of nonwoven textile fabric joined to a fibrous elastic layer are described in European Patent Application EP 0 110 010 published on April 8, 1987, inventors J. Taylor et al.. The composite nonwoven fabrics are commonly referred to as stretch bonded laminates.

In another embodiment, waistband 32 comprises a composite elastomeric web comprising individual, discrete strips or strands of elastomeric material. secured to one or more nonwoven fibrous layers. Such a composite elastomeric web may, for example, comprise an elastomeric meltblown material arranged in a selected pattern of strips and suitably sandwiched and joined between two layers of nonwoven fibrous material. This material, as well as others, is described in U.S. Patent No. 4,861,652 issued August 29, 1989. Still other useful composite elastic materials are described in U.S. 4,883,549, issued November 28, 1989.

One of the innovative features of the present invention is a continuous waistband 32 (Fig. 1) that fully gathers the partially elastic waistborder 30 of a partially elastic pant body 12 in a substantially uniform manner. Another innovative feature of the present invention is a substantially uniformly, fully gathered, elastic waistborder 30 in which non-elastic front and back segments 84, 86 have a selected elasticity. Yet another innovative feature of the present invention is a substantially uniformly, fully gathered, elastic waistborder 30 that does not substantially inhibit the elasticity of elastic side sections 20 of garment 10 when in use.

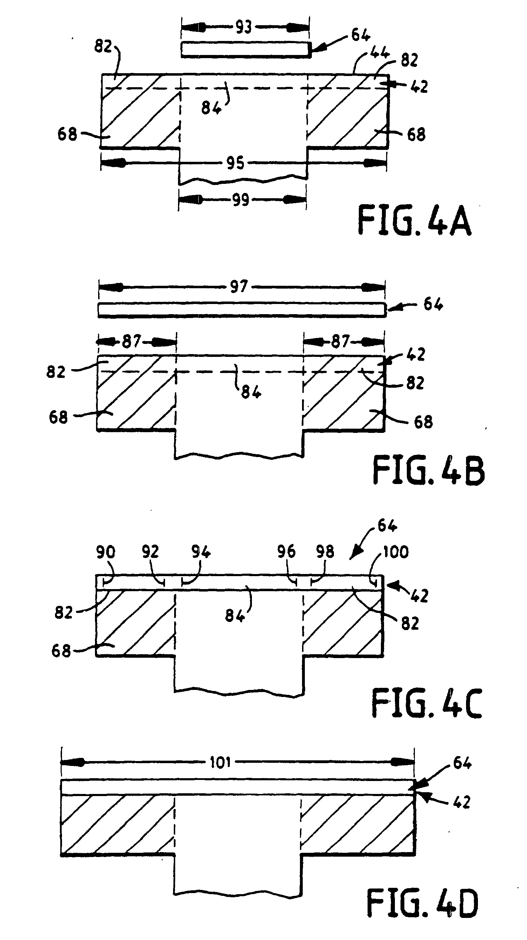

Following is one example of a substantially uniformly, fully gathered, elastic waistborder incorporated into a partially elastic pant. In this example, all lengths, elasticities, sums, differences, quotients, and products are approximate, i.e., rounded-off for ease of explanation and understanding. With reference to Figs. 3-4D, front and back waistborder sections 42, 46 (Fig. 3) define continuous waistborder 30 (Fig. 1), and have front and back waistband members 64, 66 suitably joined thereto, respectively. Since the joining of front waistband member 64 to front waistborder section 42 is identical to the joining of back waistband member 66 to back waistborder section 46, a description only of the former will be given. In this example, each elastic side member 68, as well as each elastic side segment 82 which is a portion of a respective elastic side member 68, has an elasticity of about 180%, i.e., each elastic side member 68 can be extended 2.8 times its original length. For example, a 10 centimeter long elastic string having an elasticity of 180% can be extended to 28 centimeters. Each elastic side member 68 (Fig. 4B) has a relaxed elastic length (REL) represented by arrow 87 of about 45 millimeters.

Non-elastic front segment 84 (Fig. 4A) of front waistborder section 42, which is between elastic side members 68, has essentially zero percent elasticity and a non-elastic segment length (NSL) represented by arrow 99 of about 150 mm.

Front waistband member 64 has an elasticity of about 250%, and a relaxed waistband length (RWL) represented by arrow 93 (Fig. 4A) of about 140 mm.

Front waistborder section 42 has a relaxed waistborder length (RBL) represented by arrow 95 (Fig. 4A) of about 240 mm.

Fig. 4A illustrates a relaxed front waistband member 64 in relation to front waistborder section 42 prior to their joining in accordance with the present invention. Fig. 4B illustrates waistband member 64 elongated approximately 70% to an extended waistband length (EWL) represented by arrow 97 of about 240 mm, which is also the length of relaxed waistborder length (RBL) 95 (Fig. 4A). After extending waistband member 64, it is temporarily elastically inhibited by, for example, compression. The compression can be accomplished in any suitable manner known in the art. After compression, waistband member 64 has a remaining elasticity of about 180% (250% minus 70%), which is also the elasticity of elastic side members 68 and elastic side segments 82. After extending and compressing waistband member 64, it is operatively elastically joined; by suitably intermittently joining by, for example, ultrasonic bonding, heat bonding, adhesive point bonding, or the like; to front waistborder section 42.

Fig. 4C illustrates waistband member 64 joined to waistborder section 42 at six bond locations; only six bond locations are illustrated for ease of explanation and understanding, but a greater or lesser number of bond locations can be used. Specifically, bond locations 90, 92 join waistband member 64 to one elastic side segment 82 (Fig. 4C), bond locations 94, 96 join waistband member 64 to non-elastic front segment 84, and bond locations 98, 100 join waistband member 64 to the other elastic side segment 82. For purposes of explanation, assume that waistband member 64 now is activated, such as, for example, with heat, to recover to an elasticity of about 250%. Front waistborder section 42 is then substantially uniformly, fully gathered by waistband member 64, as illustrated in Fig. 1. By "substantially uniformly" or "substantially uniform" is meant that front waistborder section 42 has one elasticity value that is substantially constant, i.e., substantially uniform, along its width, and that one elasticity value in this example is 70%. Thus, front waistborder section 42 has an elasticity of at least about 70% at any point along its width. Of course, at those points along front waistborder section 42 that coincide with elastic side segments 82 (Figs. 3, 4C), the elasticity is at least about 180%.

Waistband member 64 now can elongate only 70% between bond locations 94, 96 since non-elastic front segment 84 has, for all practical purposes herein, zero percent elasticity and is a limiting extension factor to waistband member 64. Non-elastic front segment 84 has, therefore, an elasticity the same as the elasticity of waistband member 64 between bond locations 94, 96, which is about 70%. In other words, waistband member 64 has "lost" 180% of its elasticity (250% minus 70%) between bond locations 94, 96 because of its joinder to non-elastic front segment 84.

In contrast, at elastic side segments 82, waistband member 64 has about 250% elasticity available. Upon elongating waistband member 64 to about 70%, elastic side segments 82 will be extended to their original relaxed elastic length (REL) 87 of 45 mm. Since elastic side segments 82 have an elasticity of about 180%, waistband member 64 can continue to extend its remaining elongate capacity of about 180% (250% minus 70%), as illustrated in Fig. 4D. In Fig. 4D, waistband member 64 and waistborder section 42 have a fully extended length (FEL) represented by arrow 101 of about 492 mm. The 492 millimeters (mm) is the sum of NSL (Fig. 4A), 2 times REL (Fig. 4B), and 2 times 180% elongation of REL; which is 150 mm + (2 x 45 mm) + (2 x 2.8 x 45 mm) = 492 mm.

Another innovative feature of the present invention is that continuous, partially elastic waistborder 30 (Fig. 1) not only is substantially uniformly, fully gathered about its periphery, but also can have different elasticities between non-elastic segments 84, 86 (Fig. 3) and elastic side segments 82. However, if desired, non-elastic segments 84, 86 can have an elasticity the same as the elasticity of elastic side segments 82. In the above example, this would be accomplished by providing a waistband member 64 with an elasticity of about 360%, and then elongating it 180% before temporarily elastically inhibiting and joining to waistborder sections 42. Here again, the continuous, partially elastic waistborder 30 would still be substantially uniformly, fully gathered.

In a similar manner, back waistband member 66 is joined to back waistborder section 46. Thereafter, disposable absorbent garment 10 is folded along a fold line generally parallel to transverse centerline 60, and front outer edges 50 (Fig. 3) are suitably bonded to back outer edges 58 to form seams 22. Garment 10 is then activated, such as with heat, in order to activate continuous waistband 32 (Fig. 1), which then substantially uniformly, fully gathers partially elastic, continuous waistborder 30. Once activated, continuous waistborder 30 has at any point thereon one elasticity value that is substantially constant, i.e., substantially uniform, which in the above example is an elasticity of about 70%.

It will be appreciated that the elasticity about waist opening 24 of garment 10 (Fig. 1) depends on various factors, such as the types of materials of which waistband 32 and waistborder 30 are comprised, their elastic characteristics, and the like. Thus, continuous waistband 32 will need to be carefully designed and constructed, as to materials and elasticity. For example, waistband 32 should be made of a material having a greater elasticity than the elasticity of the structure to which it is being joined, for example, partially elastic, continuous waistborder 30 of garment 10. The reason for requiring the waistband material to have a higher elasticity than the structure to which it is joined, for example, waistborder 30, is that if the waistband's elasticity is less than the elasticity of the partially elastic structure to which it is joined, then the waistband material will be a limiting extension factor to that structure. For example, if the elastic side segments 82 have an elasticity of 180%, but waistband member 64 has an elasticity of 120%, the elastic side segments 82 will only elongate as much as the waistband, i.e., 120%.

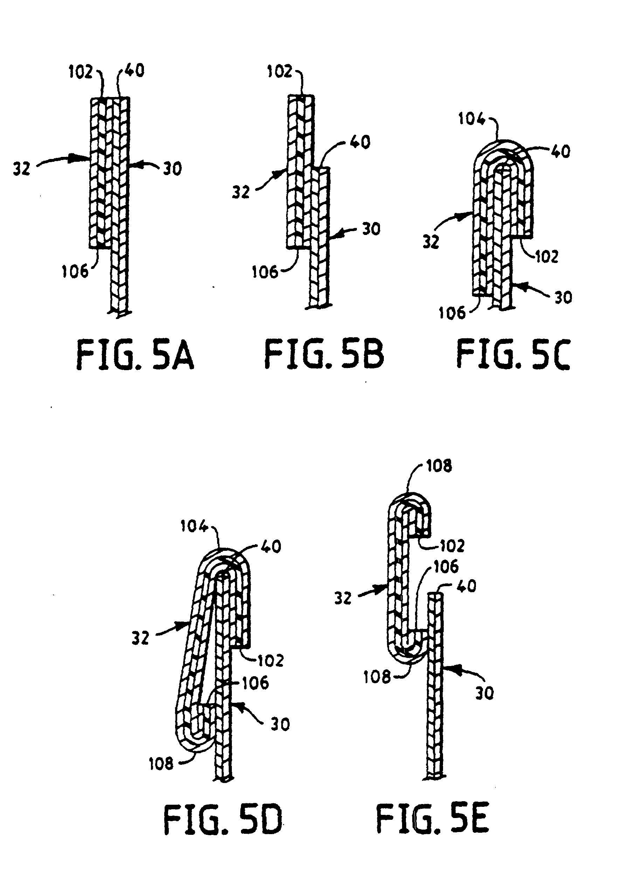

Continuous waistband 32 can be suitably joined to continuous waistborder 30 in a number of desired configurations. Fig. 5A illustrates one configuration in which continuous waistband 32 and continuous waistborder 30 are joined together such that top edge 102 of continuous waistband 32 is substantially coplanar with peripheral edge 40 of continuous waistborder 30.

Fig. 5B is similar to Fig. 5A except that continuous waistband 32 is offset from continuous waistborder 30, such that a portion of waistband 32 extends beyond continuous waistborder 30. In this configuration, top edge 102 extends beyond peripheral edge 40.

Referring to Fig. 5C, continuous waistband 32 has a folded portion 104 that is folded over peripheral edge 40, such that top edge 102 is on the side of continuous waistborder 30 opposite from bottom edge 106 of waistband 32.

The configuration in Fig. 5D is similar to that in 5C, except that the portion of continuous waistband 32 comprising bottom edge 106 has been folded upon itself to create a C-fold portion 108.

The configuration in Fig. 5E illustrates continuous waistband 32 having two C-fold portions 108, with the C-fold portion 108 that includes bottom edge 106 being suitably joined to continuous waistborder 30. In this configuration, top edge 102 extends beyond peripheral edge 40.

While this invention has been described as having a preferred embodiment, it will be understood that it is capable of further modifications. This application is therefore intended to cover any variations, equivalents, uses, or adaptations of the invention following the general principles thereof, and including such departures from the present disclosure as come or may come within known or customary practice in the art to which this invention pertains and falls within the limits of the appended claims.

## Claims

1.  A disposable garment (10), comprising:

    a pant body (12) defining a waist opening (24) and a pair of leg openings (26),
    a partially elastic, continuous waistborder (30) comprising elastic side segments (82) and at least one non-elastic segment (84, 86), and a continuous, elastic waistband (32) joined to said partially elastic, continuous waistborder (30),

**characterised in that**

said continuous, elastic waistband (32) substantially uniformly, fully gathering said partially elastic, continuous waistborder (30) without substantially inhibiting elasticities of said elastic side segments (82).

2. The garment (10) of claim 1 wherein said elastic waistband (32) provides said elastic side segments (82) and said non-elastic segment (84, 86) with different elasticities.

3. The garment (10) of claim 1 wherein said elastic waistband (32) provides said elastic side segments (82) and said non-elastic segment (84, 86) with substantially the same elasticity.

4. The garment (10) of claim 1 wherein said elastic waistband (32) is folded over a peripheral edge of said waistborder (30).

5. The garment (10) of claim 1 wherein said elastic waistband (32) is C-folded upon itself.

6. The garment (10) of claim 1 wherein said elastic waistband (32) extends beyond a peripheral edge of said waistborder (30).

7. The garment (10) of claim 1 wherein said elastic waistband (32) has a width between about 1 centimeter to about 8 centimeters.

8. The garment (10) according to claim 1 wherein said continuous waistborder (30) defines a continuous waist opening (24), wherein

said continuous elastic waistband (32) comprises a material that is capable of being temporarily elastically inhibited, and has an elasticity equal to or greater than an elasticity of said elastic segment (82), and wherein

said continuous elastic waistband (32) is joined to said continuous waistborder (30) in a temporarily elastically inhibited state.

9. The garment (10) of claim 8 wherein said non-elastic segment (84, 86), after said elastic waistband (32) has been activated, has an elasticity different from the elasticity of said elastic segment (82).

10. The garment (10) of claim 9 wherein said elastic waistband (32) has a width between about 1 centimeter to about 8 centimeters.

11. The garment (10) of claim 10 wherein said elastic waistband (32) is folded over a peripheral edge of said waistborder (30).

12. The garment (10) of claim 10 wherein said elastic waistband (32) is C-folded upon itself.

13. The garment (10) of claim 10 wherein said elastic waistband (32) extends beyond a peripheral edge of said waistborder (30).

14. The garment (10) of claim 8 wherein said non-elastic segment (84, 86) after said elastic waistband (32) has been activated, has an elasticity substantially the same as the elasticity of said elastic segment (82).

15. The absorbent garment (10) according to any one of the preceding claims, further comprising:

a topsheet, (34)
a backsheet (36) wherein the partially elastic waistborder (30) is comprised in the backsheet (36), and
an absorbent structure (28) disposed between said topsheet (34) and said backsheet (36).

16. The garment (10) of claim l5 wherein said elastic waistband (32) comprises a material that is temporarily elastically inhibited before being joined to said partially elastic waistborder (30),
said elastic waistband (32) gathering said partially elastic waistborder (30) upon being activated.

17. The garment (10) of claim 15 wherein said elastic waistband (32) is folded over an edge of said waistborder (30).

18. The garment (10) of claim 15 wherein said elastic waistband (32) extends beyond an edge of said waistborder (30).

**Patentansprüche**

1. Wegwerfbekleidungsstück (10) umfassend:

einen Hosenkörper (12), der eine Taillenöffnung (24) und ein Paar Beinöffnungen (26) begrenzt,

eine teilweise elastische, fortlaufende Taillenbegrenzung (30) umfassend elastische Seitenabschnitte (82) und wenigstens einen nichtelastischen Abschnitt (84, 86), und

ein fortlaufendes, elastisches Taillenbündchen (32), das mit der teilweise elastischen, fortlaufenden Taillenbegrenzung (30) verbunden ist, **dadurch gekennzeichnet, dass**

das fortlaufende, elastische Taillenbündchen (32) die teilweise elastische, fortlaufende Taillenbegrenzung im Wesentlichen gleichmäßig und ganz rafft, ohne die Elastizität der elastischen Seitenabschnitte (82) wesentlich zu hemmen.

2. Kleidungsstück (10) gemäß Anspruch 1, wobei das elastische Taillenbündchen (32) die elastischen Seitenabschnitte (82) und den nichtelastischen Abschnitt (84, 86) mit unterschiedlichen Elastizitäten versieht.

3. Kleidungsstück (10) gemäß Anspruch 1, wobei das elastische Taillenbündchen (32) die elastischen Seitenabschnitte (82) und den nichtelastischen Abschnitt (84, 86) mit im Wesentlichen derselben Elastizität versieht.

4. Kleidungsstück (10) gemäß Anspruch 1, wobei das elastische Taillenbündchen (32) über eine Endkante der Taillenbegrenzung (30) gefaltet ist.

5. Kleidungsstück (10) gemäß Anspruch 1, wobei das elastische Taillenbündchen (32) auf sich selbst C-gefaltet ist.

6. Kleidungsstück (10) gemäß Anspruch 1, wobei sich das elastische Taillenbündchen (32) über eine Endkante der Taillenbegrenzung (30) hinaus erstreckt.

7. Kleidungsstück (10) gemäß Anspruch 1, wobei das elastische Taillenbündchen (32) eine Breite zwischen etwa 1 Zentimeter und etwa 8 Zentimeter aufweist.

8. Kleidungsstück (10) gemäß Anspruch 1, wobei die fortlaufende Taillenbegrenzung (30) eine fortlaufende Taillenöffnung (24) begrenzt, wobei

das fortlaufende elastische Taillenbündchen (32) ein Material umfasst, welches geeignet ist, zeitweise elastisch gehemmt zu werden, und eine Elastizität aufweist, die gleich oder größer ist als eine Elastizität des elastischen Abschnittes (82), und wobei

das fortlaufende elastische Taillenbündchen (32) mit der fortlaufenden Taillenbegrenzung (30) in einem zeitweise elastisch gehemmten Zustand verbunden ist.

9. Kleidungsstück (10) gemäß Anspruch 8, wobei der nichtelastische Abschnitt (84, 86), nachdem das elastische Taillenbündchen (32) aktiviert wurde, eine Elastizität aufweist, die sich von der Elastizität des elastischen Abschnitts (82) unterscheidet.

10. Kleidungsstück (10) gemäß Anspruch 9, wobei das elastische Taillenbündchen (32) eine Breite zwischen etwa 1 Zentimeter und etwa 8 Zentimeter aufweist.

11. Kleidungsstück (10) gemäß Anspruch 10, wobei das elastische Taillenbündchen (32) über eine Endkante der Taillenbegrenzung (30) gefaltet ist.

12. Kleidungsstück (10) gemäß Anspruch 10, wobei das elastische Taillenbündchen (32) auf sich selbst C-gefaltet ist.

13. Kleidungsstück (10) gemäß Anspruch 10, wobei sich das elastische Taillenbündchen (32) über eine Endkante der Taillenbegrenzung (30) hinaus erstreckt.

14. Kleidungsstück (10) gemäß Anspruch 8, wobei der nichtelastische Abschnitt (84, 86), nachdem das elastische Taillenbündchen (32) aktiviert wurde, eine Elastizität aufweist, die im Wesentlichen gleich der Elastizität des elastischen Abschnitts (82) ist.

15. Absorbierendes Kleidungsstück (10) gemäß einem der vorhergehenden Ansprüche, ferner umfassend:

eine Vorderschicht (34),

eine Rückschicht (36), wobei die teilweise elastische Taillenbegrenzung (30) in der Rückschicht (36) umfasst ist, und

eine absorbierende Struktur (28), die zwischen der Vorderschicht (34) und der Rückschicht (36) angeordnet ist.

16. Kleidungsstück (10) gemäß Anspruch 15, wobei das elastische Taillenbündchen (32) ein Material umfasst, welches vor dem Verbinden mit der teilweise elastischen Taillenbegrenzung (30) zeitweise elastisch gehemmt ist, wobei das elastische Taillenbündchen (32) nach dem Aktiviertwerden die teilweise elastische Taillenbegrenzung (30) rafft.

17. Kleidungsstück (10) gemäß Anspruch 15, wobei das elastische Taillenbündchen (32) über eine Kante der Taillenbegrenzung (30) gefaltet ist.

18. Kleidungsstück (10) gemäß Anspruch 15, wobei sich das elastische Taillenbündchen (32) über eine Kante der Taillenbegrenzung (30) hinaus erstreckt.

## Revendications

1. Vêtement jetable (10), comprenant .

   un corps de culotte (12) définissant une ouverture de ceinture (24) et une paire d'ouvertures de jambe (26),
   une bordure de ceinture (30) continue, partiellement élastique, comprenant des segments latéraux élastiques (82) et au moins un segment non-élastique (84,86), et
   une ceinture (32) continue, élastique, réunie à ladite bordure de ceinture (30) continue, partiellement élastique, **caractérisé en ce que**
   ladite ceinture (32) continue, élastique, fronce sensiblement uniformément et totalement, ladite bordure de ceinture (30) continue, partiellement élastique, sans sensiblement inhiber les élasticités desdits segments latéraux élastiques (82).

2. Vêtement (10) selon la revendication 1, dans lequel ladite ceinture élastique (32) fournit lesdits segments latéraux élastiques (82) et ledit segment non-élastique (84,86) avec différentes élasticités.

3. Vêtement (10) selon la revendication 1, dans lequel ladite ceinture élastique (32) fournit lesdits segments latéraux élastiques (82) et ledit segment non-élastique (84,86) avec sensiblement la même élasticité.

4. Vêtement (10) selon lâ revendication 1, dans lequel ladite ceinture élastique (32) est repliée sur un bord périphérique de ladite bordure de ceinture (30).

5. Vêtement (10) selon la revendication 1, dans lequel ladite ceinture élastique (32) est repliée en C sur elle-même.

6. Vêtement (10) selon la revendication 1, dans lequel ladite ceinture élastique (32) s'étend au-delà d'un bord périphérique de ladite bordurede ceinture (30).

7. Vêtement (10) selon la revendication 1, dans lequel ladite ceinture élastique (32) a une largeur comprise entre environ un centimètre et environ huit centimètres.

8. Vêtement (10) selon la revendication 1, dans lequel ladite bordure de ceinture (30) continue définit une ouverture de ceinture (24) continue, et dans lequel

   ladite ceinture élastique (32) continue est constituée d'un matériau qui est capable d'être temporairement inhibé sur le plan de son élasticité, et a une élasticité égale ou supérieure à une élasticité dudit segment élastique (82), et

   ladite ceinture élastique (32) continue est réunie à ladite bordure de ceinture (30) continue à l'état temporairement inhibé sur le plan de son élasticité.

9. Vêtement (10) selon la revendication 8, dans lequel ledit segment non-élastique (84,86), après que ladite ceinture élastique (32) ait été activée, a une élasticité différente de l'élasticité dudit segment élastique (82).

10. Vêtement (10) selon la revendication 9, dans lequel ladite ceinture élastique (32) a une largeur comprise entre environ un centimètre et environ huit centimètres.

11. Vêtement (10) selon la revendication 10, dans lequel ladite ceinture élastique (32) est repliée sur un bord périphérique de ladite bordure de ceinture (30).

12. Vêtement (10) selon la revendication 10, dans lequel ladite ceinture élastique (32) est repliée en C sur elle-même.

13. Vêtement (10) selon la revendication 10, dans lequel ladite ceinture élastique (32) s'étend au-delà d'un bord périphérique de ladite bordure de ceinture (30).

14. Vêtement (10) selon la revendication 8, dans lequel ledit segment non-élastique (84,86), après que ladite ceinture élastique (32) ait été activée, a une élasticité sensiblement identique à l'élasticité dudit segment élastique (82).

15. Vêtement absorbant (10) selon l'une quelconque des revendications précédentes, comprenant en outre :

   une feuille supérieure (34),
   une feuille support (36) dans laquelle la bordure de ceinture (30) partiellement élastique est comprise dans ladite feuille support (36), et
   une structure absorbante (28) disposée entre ladite feuille supérieure (34) et ladite feuille support (36).

16. Vêtement (10) selon la revendication 15, dans lequel ladite ceinture élastique (32) est constituée d'un matériau qui est temporairement inhibé sur le plan de son élasticité avant d'être réunie à ladite bordure de ceinture (30) partiellement élastique,
   ladite ceinture élastique (32) fronçant ladite bordure de ceinture (30) partiellement élastique lorsqu'elle est activée.

17. Vêtement (10) selon la revendication 15, dans le-

**EP 0 955 976 B1**

quel ladite ceinture élastique (32) est pliée sur un bord de ladite bordure de ceinture (30).

18. Vêtement (10) selon la revendication 15, dans lequel ladite ceinture élastique (32) s'étend au-delà d'un bord de ladite bordure de ceinture (30).

FIG.1

FIG. 2

FIG.3

13

FIG.4A

FIG.4B

FIG.4C

FIG.4D

FIG. 5A  FIG. 5B  FIG. 5C

FIG. 5D  FIG. 5E